# EUROPEAN PATENT SPECIFICATION

(11) **EP 2 181 767 B1**
(45) Date of publication and mention of the grant of the patent: **27.06.2012**
(21) Application number: 09178039.5
(22) Date of filing: 29.05.2002
(51) Int. Cl.: B01L 3/00

(54) **Well for processing and filtering a fluid**
Vertiefung zur Verarbeitung einer Flüssigkeit
Puit pour le traitement d'un fluide

(30) Priority: 31.05.2001 US 294211 P
(43) Date of publication of application: 05.05.2010
(62) Divisional of application: 02734550.3
(73) Proprietor: Pall Corporation, Port Washington, NY 11050 (US)
(72) Inventor: Kane, Jeffrey, Manchester, MI 48158 (US)
(74) Representative: Boehme, Ulrich

(56) References cited:
- EP-A- 0 359 249
- EP-A- 1 110 611
- US-A- 4 948 442
- US-A- 6 159 368

## Description

### FIELD OF THE INVENTION

This invention relates to wells for use in processing fluids, e.g., by titration and/or filtration, and more preferably relates to multiwell microtitration and microfiltration devices.

### BACKGROUND OF THE INVENTION

Multiple well test devices are used in a variety of microtitration and/or microfiltration protocols. Typically, the devices have a standard number of wells, e.g., 96 wells, or 384 wells (arranged in four blocks of 96 wells each), and the wells include a filter arranged such that application of a vacuum or air pressure to one side of the device causes the fluid in each well to pass through the filter.

When the device is used in for microtitration, e.g., for an immunoassay or a hybridization assay, the filter is generally used to support one or more components of the assay, such as an antigen, antibody, nucleic acid probe or nucleic acid sample. A supported component (e.g., a specific binding agent) binds to an unsupported component in a fluid sample. At least one component can be contained in, on, and/or through the filter, e.g., by physical entrapment, chemical binding and/or adsorption.

When used for microfiltration the filter is generally used to remove one or more components from solutions passed through it, e.g., on the basis of physical, biological and/or chemical interactions in or on the filter. The interactions can be, for example, between the filter and the component to be retained, or between one or more materials retained in or on the filter and the component to be retained.

Accordingly, the devices can be utilized to remove one or more undesirable and/or desirable materials from a fluid. For example, the devices can be used to remove particulates from a sample before further processing (e.g., analysis) of the sample and/or to retain a desired ligand for later recovery and further processing.

Some conventional devices are labor intensive to manufacture and/or are not adapted for use with a variety of filters. Additionally, the seal of the filter in the device can be adversely affected by handling and/or fluid processing conditions.
WO 00/25922 discloses a multiple well device comprising wells as characterized in the introductory portion of claim 1. The tube comprises at its lower end a recessed section which accommodates the filter. The filter is held in place by a drip director plate which is inserted in the recessed section as well.

The present invention provides for ameliorating at least some of the disadvantages of the prior art. These and other advantages of the present invention will be apparent from the description as set forth below.

### BRIEF SUMMARY OF THE INVENTION

In accordance with the invention, a well for processing fluid comprising the features of claim 1 is provided.

Typically, the top surface of the rib arrangement is spaced away from the inner surface of the side wall. Preferably, the lower surface of the lip has a width that is at least the width of the top surface of the rib arrangement.

A well for processing fluid according to another embodiment of the invention comprises a hollow tube comprising a side wall having an inner surface and an inwardly extending lip, the lip having a rib arrangement projecting downwardly from the lip, the rib arrangement having a bottom surface; a bottom end comprising a bottom wall and a fluid flow port and a sealing ring comprising an outer surface and a top surface, the outer surface of the sealing ring pressing against the inner surface of the side wall of the tube, and a filter comprising at least one filter element, the filter having an upper surface and a lower surface, the filter being compressed between the top surface of the sealing ring and the bottom surface of the rib arrangement.

In some embodiments of the well, the bottom end also includes a drainage grid comprising at least one drainage channel communicating with the fluid flow port. For example, the bottom end can include a drainage grid comprising a plurality of drainage grid spacers projecting upwardly from the bottom wall, wherein a plurality of drainage channels are provided between the drainage grid spacers, and the drainage channels communicate with the fluid flow port.

In accordance with another embodiment, a plurality of the wells are connected together to provide a multiple well device, e.g., in the form of a 96 or 384 well device, in some embodiments, a 96 or 384 well tray.

Methods are also provided for processing a fluid utilizing embodiments of the well and the multiple well devices. Suitable methods include, for example, microtitration, microfiltration, and microculture procedures.

### BRIEF DESCRIPTION OF THE DRAWINGS

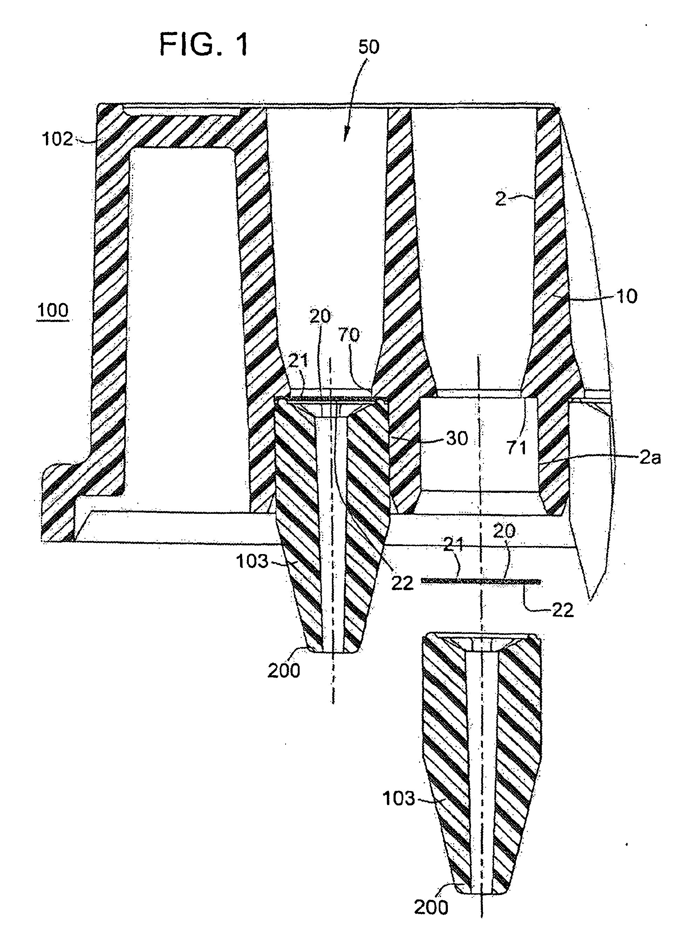

Figure 1 shows a partial cross-sectional view of another embodiment of a multiple well device according to the invention, showing an assembled well and an exploded view of a well. The Figure shows a top plate including inwardly extending lips, wherein each lip has a bottom surface; and individual lower chambers, each chamber including the bottom end of a well, wherein each bottom end includes a bottom wall, a fluid flow port, and a sealing ring comprising a rib arrangement projecting upwardly from the bottom wall, wherein the sealing ring is retained such that the outer surface of the sealing ring presses against the inner surface of the side wall of the well, and the bottom surface of the lip presses against the upper surface of the filter while the top surface of the rib arrangement presses against the lower surface of the filter, so that the filter is sealed in the well.

Figure 2 shows a cut away view of the lower chamber of one of the wells shown in Figure 1, showing the bottom end of the well, including a bottom wall, a fluid flow port, and a sealing ring comprising a rib arrangement projecting upwardly from the bottom wall.

Figure 3 shows a top view of the bottom end of the well shown in Figure 1, showing a bottom wall, a rib arrangement, a drainage grid, and a fluid flow port.

Figure 4 shows a top view of the top plate shown in Figure 1.

Figure 5 shows a partial cross-sectional view of the top plate shown in Figure 4.

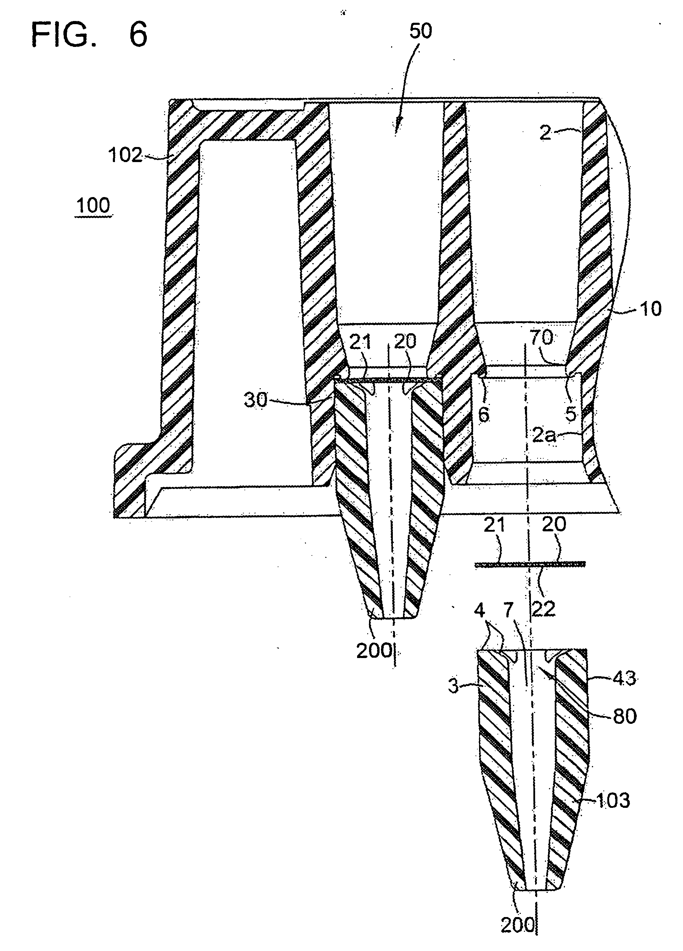

Figure 6 shows a partial cross-sectional view of another embodiment of a multiple well device according to the invention, showing an assembled well and an exploded view of a well. The Figure shows a top plate including inwardly extending lips, wherein each lip has a rib arrangement projecting downwardly from the lip; and individual lower chambers, each chamber including the bottom end of a well, wherein each bottom end includes a bottom wall, a fluid flow port, and a sealing ring having an outer surface and a top surface, wherein the sealing ring is retained such that the outer surface of the sealing ring presses against the inner surface of the side wall of the well, and the bottom surface of the rib arrangement presses against the upper surface of the filter while the top surface of the sealing ring presses against the lower surface of the filter, so that the filter is sealed in the well.

Figure 7 shows a top view of the bottom end of the well shown in Figure 6, showing a bottom wall, a drainage grid, and a fluid flow port.

Figure 8 shows a cut away view of the lower chamber of one of the wells shown in Figure 6, showing the end of the well, including a bottom wall, a fluid flow port, and a sealing ring.

Figure 9 shows a partial cross-sectional view of the top plate shown in Figure 6.

### DETAILED DESCRIPTION OF THE INVENTION

In an embodiment of the present invention, a well for use in processing a fluid comprises a hollow tube having an axis, the tube comprising a side wall having an inner surface and a lip extending inwardly from the side wall, the lip having a lower surface; upper and lower axially spaced ends, the lower end comprising a bottom wall and a fluid flow port, and a sealing ring comprising a rib arrangement projecting upwardly from the bottom wall, the rib arrangement having a top surface; the sealing ring comprising an outer surface and the top surface of the rib arrangement, the outer surface of the sealing ring pressing against the inner surface of the side wall; and a filter comprising at least one filter element, the filter having a upper surface and a lower surface, the filter being compressed between the lower surface of the lip and the top surface of the rib arrangement. Typically, the top surface of the rib arrangement is spaced from the inner surface of the side wall. Preferably, the top surface of the rib arrangement has a radial width that is at least the radial width of the lower surface of the lip. Typically, the sealing ring seals the filter against the rib arrangement wherein the top surface of the sealing ring presses against the lower surface of the filter while the lower surface of the lip presses against the upper surface of the filter.

A well for use in processing a fluid according to another embodiment of the invention comprises a hollow tube having an axis, the tube comprising a side wall having an inner surface and a lip extending inwardly from said side wall, the lip having a lower surface; upper and lower axially spaced ends, the lower end comprising a bottom wall and a fluid flow port and a sealing ring comprising an outer surface and a top surface, the outer surface of the sealing ring pressing against the inner surface of the side wall of the tube; a filter comprising at least one filter element, the filter having an upper surface and a lower surface, the filter being compressed between the top surface of the sealing ring and the lower surface of the lip. In a preferred embodiment, the lip comprises a rib arrangement projecting downwardly from the lip, more preferably, wherein the rib arrangement has a bottom surface spaced from the inner surface of the side wall, and the filter is compressed between the top surface of the sealing ring and the bottom surface of the rib arrangement. In another embodiment, the sealing ring further comprises a rib arrangement projecting upwardly from the bottom wall, the rib arrangement having a top surface, wherein the filter is compressed between the top surface of the rib arrangement and the lower surface of the lip. The top surface of the rib arrangement can be spaced from the inner surface of the side wall.

In more preferred embodiments of the invention, a plurality of wells are connected together to provide a multiple well device. In accordance with embodiments of the invention, the potential for cross-contamination from one well to another can be eliminated.

In accordance with another embodiment, a multiple well device comprises a plurality of wells for receiving liquid samples to be processed, each well comprising a side wall having an inner surface and a lip extending inwardly from the side wall, the lip having a lower surface; a bottom end comprising a bottom wall and a fluid flow port and sealing ring comprising a rib arrangement projecting upwardly from the bottom wall, the rib arrangement having a top surface that does not contact the inner surface of the side wall, the sealing ring comprising an outer surface; and a filter comprising at least one filter element, the filter having a upper surface and a lower surface; the outer surface of the sealing ring pressing against the inner surface of the side wall; and the lower surface of the lip pressing against the upper surface of the filter while the top surface of the rib arrangement presses against the lower surface of the filter.

In accordance with an embodiment of the invention, a multiple well device comprises a plurality of wells for receiving liquid samples to be processed, each well comprising a side wall having an inner surface and a lip extending inwardly from the side wall, the lip having a rib arrangement projecting downwardly from the lip, the rib arrangement having a bottom surface that does not contact the inner surface of the side wall; a bottom end comprising a bottom wall and a fluid flow port and a sealing ring comprising an outer surface and a top surface, wherein the bottom wall of the bottom end comprises the top surface of the sealing ring; and a filter comprising at least one filter element, the filter having a upper surface and a lower surface; the outer surface of the sealing ring pressing against the inner surface of the side wall; and the top surface of the sealing ring pressing against the lower surface of the filter while the bottom surface of the rib arrangement presses against the upper surface of the filter.

Another embodiment of a multiple well device according to the invention comprises a top plate comprising the upper ends of a plurality of wells; a plurality of separate inserts comprising the lower ends of the plurality of wells, each insert comprising a bottom wall and a fluid flow port; and a plurality of filters, each filter comprising at least one filter element, wherein each filter is sealed between the top plate and an insert. In some embodiments, the top plate includes a plurality of sealing rings, and in some other embodiments, each insert comprises a sealing ring.

Yet another embodiment of a multiple well device according to the invention comprises a bottom plate comprising the bottom ends of a plurality of wells, each bottom end comprising a bottom wall and a fluid flow port; a plurality of separate inserts comprising the upper ends of the plurality of wells; and a plurality of filters, each filter comprising at least one filter element, wherein each filter is sealed between the top plate and an insert. In some embodiments, the bottom plate includes a plurality of sealing rings, and in some other embodiments, each insert comprises a sealing ring.

Embodiments of multiple well devices include wells connected together in the form of, for example, a strip, disc, or tray. Multiple well devices can include integrally formed wells (e.g., formed by injection or blow molding), or the wells can be formed from a plurality of components, e.g., an upper plate, tray or insert defining the upper portion of the wells and/or the sealing ring, and a bottom plate, tray or insert defining at least the bottom wall of the wells.

The wells, and more preferably, the multiple well devices, are suitable for use with a variety of other components such as a chamber, plenum, manifold, manifold cover, or the like, e.g., for applying reduced pressure or vacuum to the wells. Accordingly, some embodiments of the wells and multiple well devices further comprise, for example, a manifold cover.

Embodiments of methods for using the wells and multiple well devices are also provided.

In one embodiment, a method for processing a fluid comprises passing a fluid into a device comprising a well comprising a side wall having an inner surface, and a lip extending inwardly from said side wall, the lip having a lower surface; and lower end comprising a bottom wall and a fluid flow port and a sealing ring, the sealing ring comprising a top surface; the well having a filter comprising at least one filter element sealed therein, the filter being compressed between a lower surface of the lip, and the top surface of the sealing ring, and, passing at least a portion of the fluid through the filter and through the fluid flow port at the bottom end of the well.

Preferably, the method comprises processing fluid in a multiple well device, wherein fluid is passed into a plurality of wells as described above.

Embodiments of the method can include binding at least one component in the fluid to be processed to at least one binding agent in the well, in some embodiments, binding at least one component in the fluid to be processed to at least one binding agent in or on the filter. For example, embodiments of the method can include antigen-antibody binding, binding a nucleic acid in a sample to a complementary nucleic acid probe, binding ligands, and binding proteins. In some embodiments, at least one binding agent is a specific binding agent such as a monoclonal antibody or a nucleic acid probe that specifically binds to a component in the fluid to be processed. Other embodiments of the method include, for example, removing particulates from the fluid to be processed, and synthesizing desired materials in the wells. If desired, embodiments of the method also include analyzing the filtrate passing through the filter and/or analyzing the bound component (in some embodiments, after cleaving the bound component from the well).

Each of the components of the invention will now be described in more detail below, wherein like components have like reference numbers.

A plurality of wells can be connected together, e.g., in the form of a strip, disc, sheet, or tray. In preferred embodiments, a plurality of wells are connected together for use in devices according to the invention. For example, Figure shows portions of embodiments of devices providing 384 well devices. In another illustrative embodiment (not shown) a plurality of wells connected in the form of one or more strips can be attached (e.g., by a snap fit) to a support, frame, or plate, Alternatively, or additionally, e.g., in a variation of the embodiments shown in Figures 1 and 6, a plurality of inserts are connected together, for example, in the form of a strip, disc, sheet, or tray.

In some embodiments of the invention, especially some embodiments of multiple well devices according to the invention, the device comprises at least one plate or tray defining at least a part of the upper portion of the wells or at least a part of the lower portion of the wells (the plate or tray can comprise the sealing ring); a plurality of separate components (preferably inserts) defining at least a part of another portion of the well (e.g., a part of the lower portion wherein the plate or tray defines at least a part of the upper portion of the wells; and the separate insert can further comprise the sealing ring), and a filter compressed by the sealing ring. In other embodiments of multiple well devices, the device comprises at least a top plate and a bottom plate, and a filter. For example, the device can comprise an upper plate or tray defining the upper portion of the wells and/or the sealing ring, a bottom plate or tray defining at least the bottom wall of the wells, and a filter between the sealing ring and the bottom wall. Typically, the plates and/or separate components are snap-fit or press-fit together. In some embodiments, the plates and/or separate components are snap-fit or press-fit together and subsequently additionally bonded, e.g., via welding (such as ultrasonic welding), adhesives and/or solvents.

In accordance with the embodiment illustrated in partial cross-sectional view in Figure 1 (showing an assembled well and an exploded view of a well), multiple well device 100 comprises a top plate 102 having a plurality of wells 50, each well comprising a side wall 10 having an inner surface 2, an inwardly extending lip 70 having a lower surface 71 (the lip, typically an annular member, e.g., a ledge or flange, and the side wall, can be injection molded as a unitary piece); and a bottom chamber 103 (preferably a separate insert, typically forming a nozzle) comprising a bottom end 3 comprising a bottom wall 4, the bottom end also comprising a sealing ring 40 comprising a rib arrangement 5 having a top surface 6 projecting upwardly from the bottom wall, a grid arrangement 80, and a fluid flow port 7; and a filter 20 comprising at least one filter element 25, compressed between the top surface 6 of the rib arrangement 5 (the top surface of the sealing ring) and the lower surface 71 of the lip.

In the embodiment illustrated in Figure 1, the sealing ring 40 comprises an outer surface, 43, and the rib arrangement 5 wherein the top surface 6 of the rib arrangement provides the top surface of the sealing ring. The top surface 6 (that is preferably a non-planar surface) is disposed to contact the lower surface 22 of the filter 20 comprising at least one filter element 25. Typically, the top surface 6 of the rib arrangement does not contact the inner surface 2 of the side wall of the well, and the rib arrangement can be located a predetermined distance from the inner surface of the side wall, e.g., a predetermined distance from that portion of the inner surface (portion 2a of inner surface 2) below the lower surface 71 of lip 70. Preferably, the top surface of the rib arrangement has a radial width that is at least the radial width of the lower surface of the lip.

In sealing the filter in the device illustrated in Figure 1, the outer surface 43 of the sealing ring presses against the inner surface 2 of the side wall, and the lower surface of the lip presses against a portion of the upper surface 21 of the filter 20 (e.g., at least the portion of the upper surface 21 opposite the portion of the lower surface of the filter contacting the top of the rib arrangement) while the top surface 6 of the rib arrangement 5 presses against the lower surface 22 of the filter. As with the other embodiments, fluid is prevented from bypassing the filter.

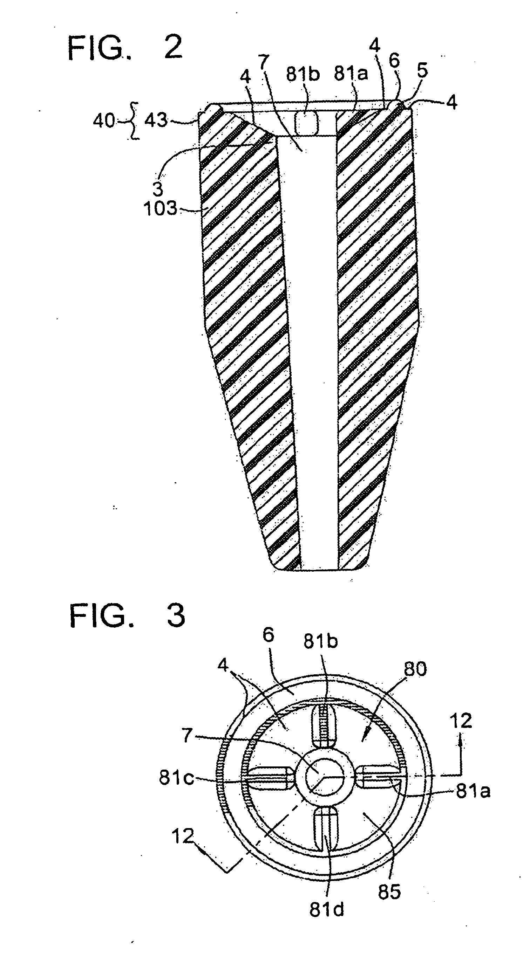

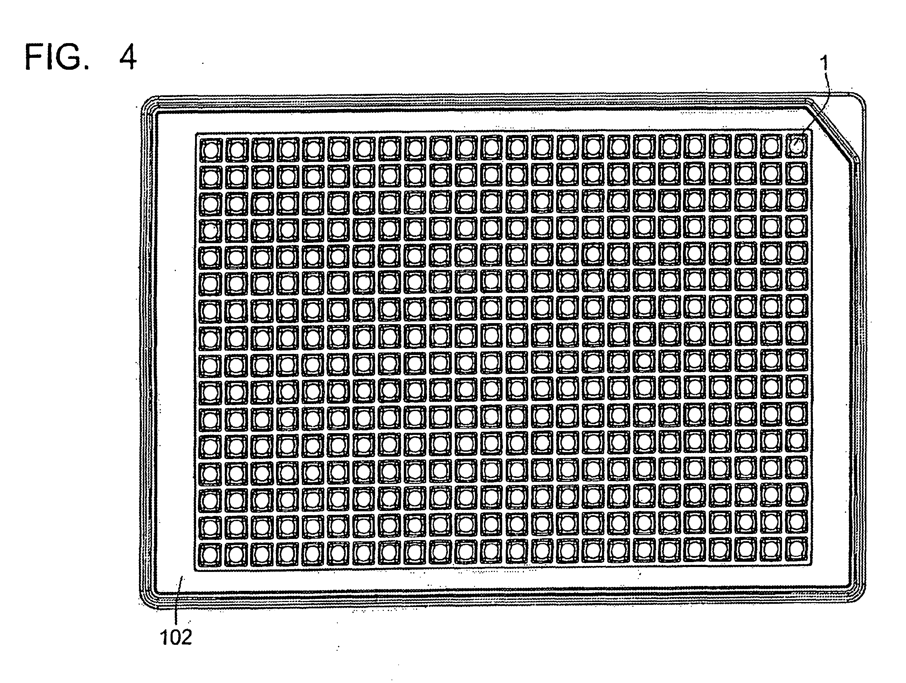

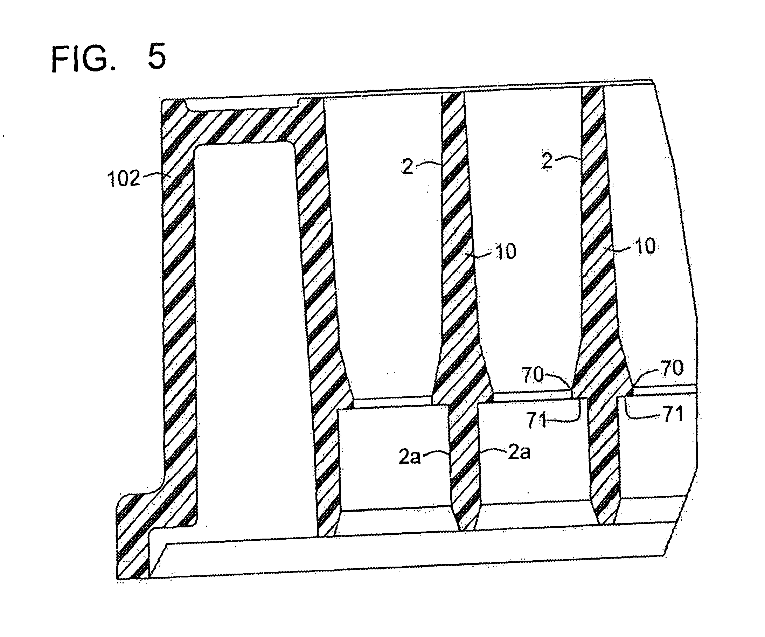

Figures 2 to 5 illustrate the individual components of the multiple well device 100 shown in Figure 1. Accordingly, Figures 2 (partial cut away view) and 3 (top view) show the bottom chamber 103, comprising the bottom end 3 including a bottom wall 4, sealing ring 40 comprising an outer surface 43 and a rib arrangement 5 projecting upwardly from the bottom wall, a drainage grid 80 comprising a plurality of drainage grid spacers 81a-d projecting upwardly from the bottom wall 4, and a fluid flow port 7. Figures 4 and 5 show, respectively, a top view, and a partial cross-sectional view of the top plate 102 shown in Figure 1.

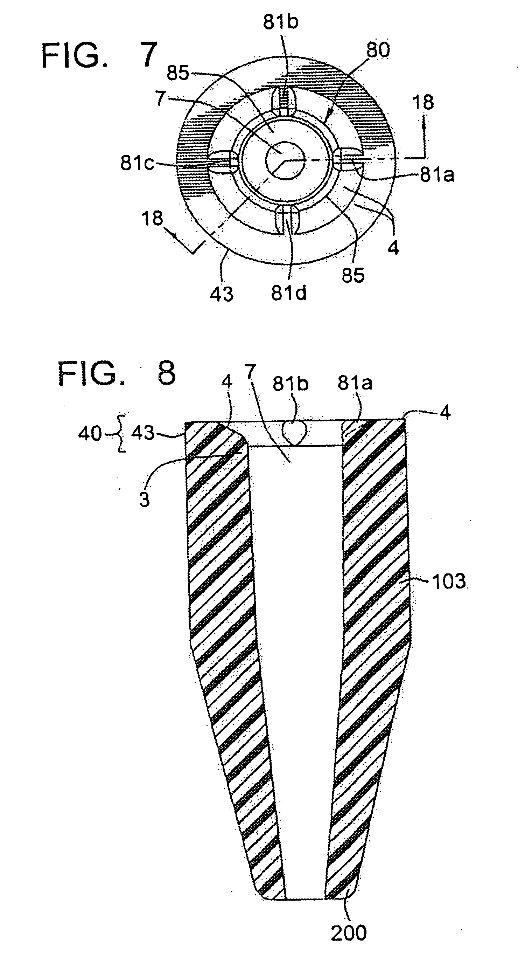

Figure 6 shows a partial cross-sectional view of another embodiment of a multiple well device according to the invention (showing an assembled well and an exploded view of a well), and Figures 7 to 9 illustrate individual components of this embodiment of the device in more detail.

Figure 6 shows multiple well device 100 comprising a top plate 102 having a plurality of wells 50, each well comprising a side wall 10 having an inner surface 2, an inwardly extending lip 70 comprising a rib arrangement 5 projecting downwardly from the lip, the lip having a bottom surface 6a, preferably spaced away from the inner surface 2 of the side wall. The lip and side wall can be injection molded as a unitary piece. Figure 16 also shows a bottom chamber 103 (preferably a separate insert, more typically forming a nozzle) comprising a bottom end 3 comprising a bottom wall 4, the bottom end also comprising a grid arrangement 80, and a fluid flow port 7, and a sealing ring 40 comprising an outer surface 43 and a top surface, wherein the bottom wall of the bottom end comprises the top surface of the sealing ring, and a filter 20 comprising at least one filter element 25, compressed between the bottom surface 6a of the rib arrangement 5 and the top surface of the sealing ring,

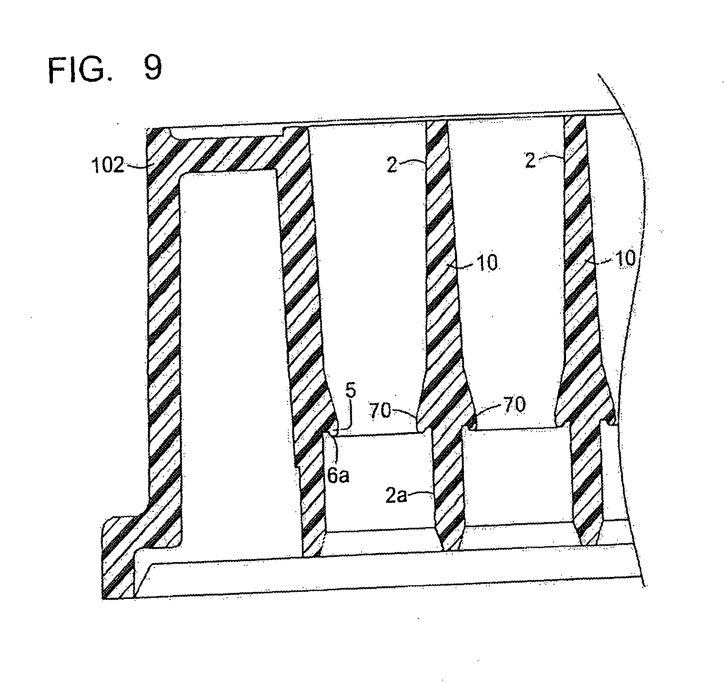

Figures 7 to 9 illustrate the individual components of the multiple well device 100 shown in Figure 6. Accordingly, Figures 7 (top view) and 8 (partial cut away view) show the bottom chamber 103, comprising the bottom end 3 including a bottom wall 4, sealing ring 40 comprising an outer surface 43 and a top surface, wherein the bottom wall of the bottom end comprises the top surface of the sealing ring, a drainage grid 80 comprising a plurality of drainage grid spacers 81a-d projecting upwardly from the bottom wall 4, and a fluid flow port 7. Figure 9 shows a partial cross-sectional view of the top plate 102 shown in Figure 6, including side wall 10 having an inner surface 2, an inwardly extending lip 70 comprising a rib arrangement 5 projecting downwardly from the lip, the lip having a bottom surface 6a. In this illustrated embodiment, the bottom surface 6a is spaced away from the inner surface 2 of the side wall (e.g., the Figure shows a depression between the bottom surface 6a and portion 2a of inner surface 2). Preferably, the bottom surface 6a of the rib arrangement has a radial width that is at least the radial width of the top surface of the sealing ring.

In sealing the filter in the device shown in Figure 6, the outer surface 43 of the sealing ring presses against the inner surface 2 of the side wall 10 (at portion 2a), and the bottom surface 6a of the rib arrangement 5 presses against a portion of the upper surface 21 of the filter 20 while the top surface of the sealing ring presses against the lower surface 22 of the filter. As with the other embodiments, fluid is prevented from bypassing the filter.

In a variation of the embodiment illustrated in Figures 7 to 9, the inwardly extending lip does not include a rib arrangement projecting downwardly from the lip. For example, the lip can have a lower surface, and the lower surface presses against a portion of the upper surface of the filter while the top surface of the sealing ring presses against the lower surface of the filter.

As described above with respect to embodiments of the invention, the sealing ring 40 is preferably frictionally engaged against the inner wall of the well, while providing for sealing the filter in the well without requiring the use of welding and/or adhesives to retain the ring in the well. The sealing ring can be frictionally engaged against the wall at position 30 as shown in the embodiments illustrated in Figures 1 and 6. If desired, position 30 can be predetermined, e.g., by adjusting the thickness and/or density of the filter elements. Alternatively, or additionally, the position can be predetermined by controlling the force applied to introduce the inserts into the trays (e.g., introducing the inserts comprising sealing rings into the top plates using Figures 1 and 6 for reference). The frictional engagement is preferably accomplished by adjusting the relative sizes of the outer diameter of the sealing ring and the inner diameter of the well.

A variety of materials are suitable for producing rib arrangements, lips, drainage grids, inserts, chambers, plates, and sealing rings according to the invention. Illustrative materials include, for example, polyvinyl chloride with or without copolymers, polyethylenes, polystyrenes, polystyrene-acrylonitrile, polypropylene, polyvinylidine chloride, and the like.

The wells and multiple well devices according to the invention can have any suitable overall dimension and capacity, although preferred embodiments have substantially the same overall dimension and capacity of standard wells and devices. Such embodiments are preferred as being more easily utilized with devices and instruments, such as liquid handling systems and readers, that are commonly available for use with conventional wells and devices. In those applications wherein a signal is generated, e.g., in the course of a microtitration assay, the signal can be read by any suitable means, e.g., by visual analysis, or the detection of a fluorometric, spectrophotometric, radiometric, or chemiluminescent signal.

Typically, each well is suitable for receiving at least about 200 microliters (µL), more typically, at least about 350 µL, of fluid to be processed. In some embodiments, each well is suitable for receiving at least about 800 µL of fluid, or at least about 1 mL, or at least about 2 mL of fluid, or more.

If desired, the well, more typically, a multiple well device, is chemically resistant, and can be used with harsh solvents and/or harsh chemicals.

The present invention is useful in a variety of applications including microtitration, microchromatography, radiography, microfiltration, ultrafiltration, nanofiltration, washing processes, polymerase chain reaction (PCR) analysis, high throughput, especially high throughput screening (HTS), combinatorial chemistry, nucleic acid and protein processing (including synthesis, sequencing, separation and/or purification) and microculture of cell suspensions and tissues. The invention can be used in any suitable setting, including, but not limited to, hospitals and laboratories. Embodiments of the invention are suitable for a variety of protocols, including sample preparation, clinical diagnostic assays, and screening specimens, e.g., drugs in pharmaceutical research. If desired, materials (e.g., ligands, nucleic acids) can be bound to solid phase particles such as beads and collected in the wells, and the materials can be cleaved from the particles such that the materials are collected in the filtrate. In some embodiments, the cleaved materials (e.g., ligands and synthesized nucleic acids) can be further processed (e.g., screened) using another well, e.g., the filtrate can be passed into one or more multiple well devices. Alternatively, or additionally, the invention can be compatible with other subsequent processes including, but not limited to, at least one of dot blotting, immunoblotting, receptor binding assays, ELISA, and RIA.

Embodiments of the invention are especially suitable for removing particulates from a fluid, e.g., to provide a filtered sample for analysis, for example, by high pressure liquid chromatography (HPLC); gas chromatography (GC), mass spectrometry (MS), infra red (IR), nuclear magnetic resonance (NMR), and solid-phase extraction.

Accordingly, a variety of filters and filter elements (as used herein, the terms "filter" and "filter element" refer to porous media used in these various applications) are suitable for use in the invention, and those skilled in the art will recognize that the choice of filter(s) and filter element(s) will depend on the intended use of the well. The filter can comprise a depth filter and/or a sieve filter. The filter can include fibrous and/or membrane filter elements. The filter can include additional elements and/or components such as, for example, at least one of a drainage, cushion, and prefilter layer. Typical filter elements include membranes, especially polymeric membranes. For some applications, the filter elements are chemically resistant, and can be used with harsh solvents and/or harsh chemicals. In some embodiments, the filter comprises a plurality of filter elements, and the elements can have different characteristics, e.g., at least one of pore size, chemistry (for example, at least one of critical wetting surface tension, surface charge, polarity, hydrophilicity, and attached functional groups), and can include different reagents and assay components.

Filters and filter elements of suitable shape for use in the invention are typically stamped or otherwise cut out of sheets of suitable material(s), e.g., membrane sheets.

A filter element such as a porous membrane or a fibrous element can have any suitable pore structure such as a pore size, or a pore rating or a pore diameter. Thus, e.g., a filter element comprising a membrane typically has an average pore size of about 100 µm or less, preferably from about 0.01 µm to about 100 µm. In some embodiments, the membrane has an average pore size of about 0.1 µm or less, or from about 0.1 µm to about 10 µm. Preferably, the membrane has an average pore size of about 5 µm or less.

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and similar referents in the context of describing the invention (especially in the context of the following embodiments) are to be construed to cover both the singular and the plural, unless otherwise indicated herein or clearly contradicted by context. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise embodimented. No language in the specification should be construed as indicating any non-embodimented element as essential to the practice of the invention.

## Claims

1. A well (50) for use in processing a fluid comprising:
a hollow tube having an axis, the tube comprising a side wall (10) having an inner surface (2);
upper and lower axially spaced ends;
the lower end comprising a bottom wall and a fluid flow port and a sealing ring (40), the sealing ring (40) comprising an outer surface and a top surface;
a filter (20) comprising at least one filter element, the filter having an upper surface and a lower surface,
**characterized in that**
it further comprises a lip (70) extending inwardly from said side wall (10), the lip (70) having a lower surface (71);
the filter (20) being compressed between the top surface of the sealing ring (40) and the lower surface of the lip (70); and
the outer surface of the sealing ring (40) presses against the inner surface of the side wall (10) of the tube.

2. The well of claim 1, wherein the sealing ring (40) provides for said bottom wall and further comprises a rib arrangement (5) projecting upwardly from the bottom wall, the rib arrangement (5) having a top surface (6); wherein the filter (20) is compressed between the top surface of the rib arrangement (5) and the lower surface of the lip (70).

3. The well of claim 2, wherein the top surface (6) of the rib arrangement (5) is spaced from the inner surface (2) of the side wall (10).

4. The well of claim 1, wherein the lip (70) further comprises a rib arrangement (5) projecting downwardly from the lip (70), the rib arrangement (5) having a bottom surface (6), and the filter (20) is compressed between the top surface of the sealing ring (40) and the bottom surface of the rib arrangement (5).

5. The well of claim 4, wherein the bottom surface of the rib arrangement (5) is spaced from the inner surface of the side wall (10).

6. The well of any of claims 1 to 5, wherein the lower end comprises an insert.

7. The well of any of claims 1 to 6, wherein the filter (20) comprises a membrane.

8. The well of claim 7, wherein the membrane comprises a microporous membrane.

9. The well of claim 8, wherein the membrane comprises an ultrafiltration membrane.

10. The well of any of claims 1 to 6, wherein the filter (20) comprises a fibrous medium.

11. The well of any of claims 1 to 10, wherein the filter (20) comprises at least two filter elements.

12. The well of any of claims 1 to 11, wherein the side wall (10) is not tapered where the sealing ring (40) presses against the inner surface of the side wall (10).

13. A multiple well device (100) comprising:
two or more wells (50) according to any one of claims 1 to 12, preferably comprising 96 wells or 384 wells.

14. A method for processing a fluid comprising:
passing a fluid into a device comprising a well (50) according to any one of claims 1 to 12; and,
passing at least a portion of the fluid through the filter (20) and through the fluid flow port at the bottom end of the well (50).

15. The method of claim 14, wherein passing the fluid through the filter (20) includes depleting the fluid of particulates, optionally further comprising analyzing the fluid passing through the filter (20).

## Patentansprüche

1. Vertiefung (50) zur Verwendung bei der Behandlung eines Fluids, umfassend:
ein hohles Röhrchen mit einer Achse, wobei das Röhrchen eine Seitenwand (10) mit einer inneren Oberfläche (2) aufweist;
ein oberes und ein unteres Ende, welche axial voneinander beabstandet sind;
wobei das untere Ende eine Bodenwand und eine Fluiddurchflussöffnung und einen Dichtring (40) umfasst, wobei der Dichtring (40) eine äußere Oberfläche und eine obere Oberfläche umfasst;
ein Filter (20), umfassend mindestens ein Filterelement, wobei das Filter eine obere Oberfläche und eine untere Oberfläche aufweist,
**dadurch gekennzeichnet, dass**
sie ferner eine Lippe (70) umfasst, welche sich von der Seitenwand (10) einwärts erstreckt, wobei die Lippe (70) eine untere Oberfläche (71) aufweist;
wobei das Filter (20) zwischen der oberen Oberfläche des Dichtrings (40) und der unteren Oberfläche der Lippe (70) komprimiert ist; und
wobei die äußere Oberfläche des Dichtrings (40) gegen die innere Oberfläche der Seitenwand (10) des Röhrchens drückt.

2. Vertiefung nach Anspruch 1, wobei der Dichtring (40) die Bodenwand bereitstellt und ferner eine von der Bodenwand nach oben ragende Rippenanordnung (5) umfasst, wobei die Rippenanordnung (5) eine obere Oberfläche (6) aufweist;
wobei das Filter (20) zwischen der oberen Oberfläche der Rippenanordnung (5) und der unteren Oberfläche der Lippe (70) komprimiert ist.

3. Vertiefung nach Anspruch 2, wobei die obere Oberfläche (6) der Rippenanordnung (5) von der inneren Oberfläche (2) der Seitenwand (10) beabstandet ist.

4. Vertiefung nach Anspruch 1, wobei die Lippe (70) ferner eine von der Lippe (70) nach unten ragende Rippenanordnung (5) umfasst, wobei die Rippenanordnung (5) eine untere Oberfläche (6) aufweist und wobei das Filter (20) zwischen der oberen Oberfläche des Dichtrings (40) und der unteren Oberfläche der Rippenanordnung (5) komprimiert ist.

5. Vertiefung nach Anspruch 4, wobei die untere Oberfläche der Rippenanordnung (5) von der inneren Oberfläche der Seitenwand (10) beabstandet ist.

6. Vertiefung nach einem der Ansprüche 1 bis 5, wobei das untere Ende einen Einsatz umfasst.

7. Vertiefung nach einem der Ansprüche 1 bis 6, wobei das Filter (20) eine Membran umfasst.

8. Vertiefung nach Anspruch 7, wobei die Membran eine mikroporöse Membran umfasst.

9. Vertiefung nach Anspruch 8, wobei die Membran eine Ultrafiltrationsmembran umfasst.

10. Vertiefung nach einem der Ansprüche 1 bis 6, wobei das Filter (20) ein faseriges Medium umfasst.

11. Vertiefung nach einem der Ansprüche 1 bis 10, wobei das Filter (20) mindestens zwei Filterelemente umfasst.

12. Vertiefung nach einem der Ansprüche 1 bis 11, wobei die Seitenwand (10) dort, wo der Dichtring (40) gegen die innere Oberfläche der Seitenwand (10) drückt, nicht verjüngt ist.

13. Vorrichtung (100) mit mehreren Vertiefungen, umfassend:
zwei oder mehr Vertiefungen (50) nach einem der Ansprüche 1 bis 12, vorzugsweise umfassend 96 Vertiefungen oder 384 Vertiefungen.

14. Verfahren zum Behandeln eines Fluids, umfassend:
Einleiten eines Fluids in eine Vorrichtung, welche eine Vertiefung (50) nach einem der Ansprüche 1 bis 12 umfasst; und
Hindurchleiten mindestens eines Teils des Fluids durch das Filter (20) und durch die Fluiddurchflussöffnung am bodenseitigen Ende der Vertiefung (50).

15. Verfahren nach Anspruch 14, wobei das Hindurchleiten des Fluids durch das Filter (20) das Verarmen des Fluids an Partikeln umfasst, optional ferner umfassend das Analysieren des Fluids, welches durch das Filter (20) hindurchgeleitet wird.

## Revendications

1. Puits (50) pour utiliser pour le traitement d'un fluide comprenant :
un tube creux ayant un axe, le tube comprenant une paroi latérale (10) ayant une surface intérieure (2) ;
des extrémités supérieure et inférieure espacées axialement ;
l'extrémité inférieure comprenant une paroi de fond et un orifice d'écoulement de fluide et une bague d'étanchement (40), la bague d'étanchement (40) comprenant une surface extérieure et une surface supérieure ;
un filtre (20) comprenant au moins un élément filtrant, le filtre ayant une surface supérieure et une surface inférieure,
**caractérisé en ce que**
il comprend en outre une lèvre (70) s'étendant vers l'intérieur depuis ladite paroi latérale (10), la lèvre (70) ayant une surface inférieure (71);
le filtre (20) étant comprimé entre la surface supérieure de la bague d'étanchement (40) et la surface inférieure de la lèvre (70) ; et
la surface extérieure de la bague d'étanchement (40) presse contre la surface intérieure de la paroi latérale (10) du tube.

2. Puits selon la revendication 1, dans lequel la bague d'étanchement (40) forme ladite paroi de fond et comprend en outre un agencement de nervure (5) faisant saillie vers le haut depuis la paroi de fond, l'agencement de nervure (5) ayant une surface supérieure (6) ;
dans lequel le filtre (20) est comprimé entre la surface supérieure de l'agencement de nervure (5) et la surface inférieure de la lèvre (70).

3. Puits selon la revendication 2, dans lequel la surface supérieure (6) de l'agencement de nervure (5) est espacée de la surface intérieure (2) de la paroi latérale (10).

4. Puits selon la revendication 1, dans lequel la lèvre (70) comprend en outre un agencement de nervure (5) faisant saillie vers le bas depuis la lèvre (70), l'agencement de nervure (5) ayant une surface de fond (6), et le filtre (20) est comprimé entre la surface supérieure de la bague d'étanchement (40) et la surface de fond de l'agencement de nervure (5).

5. Puits selon la revendication 4, dans lequel la surface de fond de l'agencement de nervure (5) est espacée de la surface intérieure de la paroi latérale (10).

6. Puits selon l'une quelconque des revendications 1 à 5, dans lequel l'extrémité inférieure comprend un insert.

7. Puits selon l'une quelconque des revendications 1 à 6, dans lequel le filtre (20) comprend une membrane.

8. Puits selon la revendication 7, dans lequel la membrane comprend une membrane microporeuse.

9. Puits selon la revendication 8, dans lequel la membrane comprend une membrane d'ultrafiltration.

10. Puits selon l'une quelconque des revendications 1 à 6, dans lequel le filtre (20) comprend un média fibreux.

11. Puits selon l'une quelconque des revendications 1 à 10, dans lequel le filtre (20) comprend au moins deux éléments de filtre.

12. Puits selon l'une quelconque des revendications 1 à 11, dans lequel la paroi latérale (10) n'est pas effilée quand la bague d'étanchement (40) presse contre la surface intérieure de la paroi latérale (10).

13. Dispositif à puits multiples (100) comprenant :
deux ou plus puits (50) selon l'une quelconque des revendications 1 à 12, de préférence comprenant 96 puits ou 384 puits.

14. Procédé de traitement d'un fluide comprenant :
de faire passer un fluide dans un dispositif comprenant un puits (50) selon l'une quelconque des revendications 1 à 12 ; et,
de faire passer au moins une partie du fluide à travers le filtre (20) et à travers l'orifice d'écoulement de fluide à l'extrémité inférieure du puits (50).

15. Procédé selon la revendication 14, dans lequel faire passer le fluide à travers le filtre (20) inclut de débarrasser le fluide de ses particules, comprenant en outre d'analyser le fluide qui passe à travers le filtre (20).
